# EUROPEAN PATENT SPECIFICATION

(11) **EP 3 010 518 B1**
(45) Date of publication and mention of the grant of the patent: **10.06.2020**
(21) Application number: 14732604.5
(22) Date of filing: 18.06.2014
(51) Int. Cl.: A61K 35/76, A61K 38/48, A61K 31/135, A61P 35/00

(54) **VACCINIA VIRUS FOR GENE-DIRECTED ENZYME PRODRUG THERAPY**
VACCINIA-VIRUS FÜR GENGERICHTETE ENZYM-PRODRUG-THERAPIE
VIRUS VACCINAL POUR THÉRAPIE PAR PRODROGUE ACTIVÉE PAR DES ENZYMES EXPRIMÉES PAR DES GÈNES

(30) Priority: 19.06.2013 GB 201310917
(43) Date of publication of application: 27.04.2016
(73) Proprietor: The Institute of Cancer Research: Royal Cancer Hospital, London SW7 3RP (GB)
(72) Inventor: MARAIS, Richard, Manchester M20 4BX (GB); SPRINGER, Caroline, Sutton SM2 5NG (GB); TOMMASINI, Serena, London SW7 3RP (GB)
(74) Representative: Mewburn Ellis LLP
(86) International application number: PCT/GB2014/051873
(87) International publication number: WO 2014/202977

(56) References cited:
- EP-A2- 2 028 270
- WO-A1-01/85960
- WO-A2-00/66752
- WO-A2-2008/100292
- CHEN N G ET AL: "Oncolytic vaccinia virus: A theranostic agent for cancer", FUTURE VIROLOGY, FUTURE MEDICINE LTD, UK, vol. 5, no. 6, 1 November 2010 (2010-11-01), pages 763-784, XP009160290, ISSN: 1746-0794, DOI: 10.2217/FVL.10.58
- DOUGLAS HEDLEY ET AL: "Carboxypeptidase G2-based gene-directed enzyme-prodrug therapy: a new weapon in the GDEPT armoury", NATURE REVIEWS CANCER, vol. 7i, no. 11, 1 November 2007 (2007-11-01), pages 870-879, XP055140114, ISSN: 1474-175X, DOI: 10.1038/nrc2247
- SCHEPELMANN SILKE ET AL: "Systemic gene-directed enzyme prodrug therapy of hepatocellular carcinoma using a targeted adenovirus armed with carboxypeptidase G2.", CANCER RESEARCH 15 JUN 2005, vol. 65, no. 12, 15 June 2005 (2005-06-15) , pages 5003-5008, XP002729728, ISSN: 0008-5472
- CAROLINE J. SPRINGER ET AL: "Novel prodrugs which are activated to cytotoxic alkylating agents by carboxypeptidase G2", JOURNAL OF MEDICINAL CHEMISTRY, vol. 33, no. 2, 1 February 1990 (1990-02-01), pages 677-681, XP055140126, ISSN: 0022-2623, DOI: 10.1021/jm00164a034
- FRANCIS R J ET AL: "A phase I trial of antibody directed enzyme prodrug therapy (ADEPT) in patients with advanced colorectal carcinoma or other CEA producing tumours", BRITISH JOURNAL OF CANCER, NATURE PUBLISHING GROUP, GB, vol. 87, no. 6, 9 September 2002 (2002-09-09), pages 600-607, XP002466032, ISSN: 0007-0920, DOI: 10.1038/SJ.BJC.6600517
- WEBLEY S D ET AL: "Measurement of the critical DNA lesions produced by antibody directed enzyme prodrug therapy (ADEPT)", BRITISH JOURNAL OF CANCER, vol. 80, no. SUPPL. 2, July 1999 (1999-07) , page 47, XP002729729, & JOINT MEETING OF THE BRITISH ASSOCIATION FOR CANCER RESEARCH, THE BRITISH ONCOLOGICAL ASSOCIATION, T; EDINBURGH, SCOTLAND, UK; JULY 11-14, 1999 ISSN: 0007-0920

## Description

### Field of the Invention

The present invention relates to vaccinia viruses for gene-directed prodrug therapy and their use in treating tumours.

### Background of the Invention

Gene-directed enzyme prodrug therapy (GDEPT) and virally directed enzyme prodrug therapies (VDEPT (Huber et al., Proc. Natl. Acad. Sci. 91, 8302-8306, 1994)) are suicide-gene therapy approaches that aim to increase the delivery of toxic metabolites to solid tumours. This aims to overcome one of the major problems associated with current therapies for cancer, i.e., the lack of specificity, resulting in harmful side effects to normal tissues, such as the gut lining and bone marrow. The term 'GDEPT' is used to include both the viral and non-viral delivery systems. In the first step, a gene encoding a foreign, prodrug-activating enzyme is delivered to tumour cells in such a fashion as to ensure its tumour-restricted expression. Subsequent systemic administration of an appropriate prodrug results in generation of toxic metabolites only at the tumour site and consequently tumour selective killing.

The GDEPT system developed by the present inventors uses expression of the enzyme carboxypeptidase G2 (CPG2) from Pseudomonas strain RS16 (WO 96/03151). CPG2 activates prodrugs, such as benzoic acid nitrogen mustard prodrugs and phenol nitrogen mustard prodrugs, to release L-glutamic acid and an active cytotoxic drug.

Non-viral gene delivery methods have been used to express the prodrug-activating enzyme in tumour cells in vitro (Marais et al., Cancer Res. 56, 4735-4942, 1996; Chen et al., Cancer Res. 55, 581-589, 1995) and various studies have shown that viral gene therapy can be used to achieve tumour-targeted expression of the prodrug-activating enzyme in vivo (Schepelmann et al., Cancer Res. 67, 4949-4955, 2007; Hedley et. al., Nat. Rev. Can. 7, 870-879, 2007). Both non-replicating viral vectors and conditionally replicating oncolytic viruses with tumour selectivity have been used as vectors in GDEPT; non-replicating retrovirus and conditionally replicating adenovirus have been used as vectors in GDEPT clinical trials (Hedley et. al., Nat. Rev. Can. 7, 870-879, 2007) .

Most viruses have one or more genes which enhance viral replication, termed virulence genes. Wild-type adenoviruses express several proteins which disrupt cell cycle regulators in normal cells in order to promote cell division, thus creating an intracellular environment that supports the adenoviral lifecycle. For example, adenoviral E1A binds the retinoblastoma tumour suppressor protein, and E1B sequesters p53 to promote cell division and virus replication. Hence, adenoviral vectors in which the E1B gene is mutated are selective for cells where p53 is non-functional, e.g. many cancer cells. Termed replication-conditional oncolytic adenovirus, such vectors have been used to deliver a wide selection of prodrug-activating enzymes to cancer cells in vivo (Jounaidi et al., Cur. Cancer Drug Targets. 7(3): 285-301, 2007).

Suicide gene therapy of human colon carcinoma xenografts using an oncolytic adenovirus expressing CPG2, denoted AdV.hTERT-CPG2, has been previously presented (Schepelmann et al., Cancer Res. 67, 4949-4955, 2007). Nude mice with human colon carcinoma xenografts were treated with a single systemic administration of AdV.hTERT-CPG2 followed by six doses of the prodrug ZD2767P administered by injection at weekly intervals was shown to achieve significant antitumour efficacy. AdV.hTERT-CPG2 expresses E1A under the control of the human telomerase promoter, allowing it to replicate and express CPG2 in telomerase-positive tumour cells. A direct correlation between viral cytotoxicity and CPG2 production was shown in each colorectal carcinoma cell line that was tested. Whilst other studies had found prodrug activation to abolish the replication of vaccinia virus vectors used for GDEPT-type systems (Puhlmann et al., Hum. Gene Therapy, 10, 649-57, 1999; McCart et al., Gene Therapy, 7, 1217-23, 2000), Schepelmann et al. found that the adenoviral vector AdV.hTERT-CPG2 was found to replicate more effectively in tumours in animals that had received the prodrug, compared with the tumours in animals that had not received the prodrug.

WO08/100292 discloses modified vaccinia virus strains for use in diagnostic and therapeutic methods. WO00/66752 discloses GDEPT using posttranslational glycosylphosphatidylinositol addition to a prodrug activating enzyme to enable anchorage of the enzyme at the cell surface. EP2028270A2 discloses modified recombinant vaccinia viruses and uses thereof.

Despite progress in the field, adenovirus-based cancer therapies have achieved only limited therapeutic potency in clinical trials. It remains a problem in the art to provide further effective anticancer therapies.

### Summary of the Invention

Broadly, the present invention is based on the unexpected finding that vaccinia viruses that are modified to express a prodrug-activating enzyme are capable of eliciting a more potent antitumour effect than the oncolytic adenoviral vector AdV.hTERT-CPG2. Vaccinia virus, which is a member of the poxvirus family, is a highly immunogenic DNA virus that was used to eradicate smallpox. Like adenovirus, poxviruses express virulence genes which can disrupt cell cycle in normal cells in order to transform cells to promote cell division. Vaccinia virus and other poxviruses, such as myxoma, have a degree of natural affinity for tumour cells due to the dysregulation of the cell cycle in tumour cells, and this selectivity for tumour cells can be enhanced by deleting certain poxvirus virulence genes. For example, vaccinia tumour selectivity can be increased by the inactivation of virulence genes, e.g. the deletion of the viral thymidine kinase gene (Hedley et. al., Nat. Rev. Can. 7, 870-879, 2007). The present inventors have developed a modified vaccinia virus which expresses a prodrug-activating enzyme for use in a GDEPT system for treating tumours.

The invention is defined by the appended claims. Accordingly, in a first aspect, the present invention provides a vaccinia virus for use in treating a tumour in a subject,
wherein the vaccinia virus expresses a glutamate carboxypeptidase within a cell,
wherein the method includes the administration of a prodrug to the subject, wherein the prodrug is capable of being converted into an active form by the glutamate carboxypeptidase.

The vaccinia virus may be genetically modified to have enhanced tumour selectivity. The genetic modification may take the form of an inactivation mutation of one or more vaccinia virulence genes e.g., a deletion, an insertion or a substitution mutation. The inactivated virulence gene may include inactivated vaccinia virus thymidine kinase (vTK). The vaccinia virus may have additional virulence genes inactivated, e.g., the deletion of vaccinia growth factor (VGF).

vTK may be inactivated by virtue of the insertion of a CPG2 expression cassette, which both expresses CPG2 or a mutant, variant or homologue thereof, and also inactivates the vTK gene by insertion mutation. vTK may additionally comprise a luciferase expression cassette insert. When both are present, the CPG2 expression cassette and the luciferase expression cassette may be adjacent. Preferably, the CPG2 expression cassette and the luciferase expression cassette are adjacent to each other with their respective transcriptional promoters adjacent and priming in opposite directions.

Alternatively, a CPG2 expression cassette may be inserted elsewhere in the vaccinia virus genome, e.g. in another virulence gene.

The tumour to be treated may be a solid tumour. The tumour may be a head and neck cancer, colorectal cancer, lung cancer, melanoma, or breast cancer, cervix cancer, CNS cancer, ovarian cancer, kidney cancer, leukemia, brain tumour, or prostate cancer. Preferably, the tumour is selected from head and neck cancer, colorectal cancer, lung cancer, melanoma, or breast cancer.

The volume of the tumour treated according to the invention will preferably decrease following administration of the prodrug. Preferably, the subject has a chance of survival which will increase following administration of the prodrug.

The vaccinia virus may be administered to the subject by intravenous or intratumoural injection. The treatment may include the administration of radiotherapy to the subject. Further anticancer agents may also be administered to the subject, e.g. Chk1 inhibitors.

The glutamate carboxypeptidase may comprise CPG2 or a mutant, variant or homologue thereof. The CPG2 may have an amino acid sequence which is at least 80%, at least 90%, at least 95%, at least 98%, or at least 99%, or 100% identical to SEQ ID NO:2.

The prodrug may comprise a glutamic acid group or a glutamic acid analogue group, the glutamic acid group or the glutamic acid analogue being connected via N to a linker which is connected to a cytotoxic agent. The linker may comprise a functional group selected from: carbonyl, carbamate, urea, and equivalents. The cytotoxic agent may comprise a nitrogen mustard or a nitrogen mustard analogue. The prodrug may be N-(4-[bis(2-iodoethyl)amino]phenoxycarbonyl)-L-glutamic acid or a salt thereof.

In a further aspect, the invention provides a vaccinia virus which expresses a glutamate carboxypeptidase within a cell, wherein the glutamate carboxypeptidase is CPG2 which is at least 80%, at least 90%, at least 95%, at least 98%, or at least 99%, or 100% identical to SEQ ID NO:2. The CPG2 gene may be expressed by a CPG2 expression cassette inserted into a vaccinia virus virulence gene. The vaccinia virus virulence gene may be vaccinia thymidine kinase (vTK), thereby effecting inactivation of vTK. The vaccinia virus may have additional virulence genes inactivated, e.g., the deletion of vaccinia growth factor (VGF).

In another aspect, the invention provides a two component system for gene directed enzyme prodrug therapy which comprises: (a) a vaccinia virus which expresses a glutamate carboxypeptidase, and (b) a prodrug which can be converted into an active drug by a glutamate carboxypeptidase.

In yet another aspect, the invention provides an *in vitro* method of killing a neoplastic cell, the method comprising treating the neoplastic cell with: a vaccinia virus which expresses a glutamate carboxypeptidase; and, a prodrug which is capable of being converted into an active form by a glutamate carboxypeptidase.

This disclosure also provides a method of treating a tumour in a patient in need of treatment by administering to the patient: a vaccinia virus which is capable of expressing a glutamate carboxypeptidase in a cell; and, a prodrug which is capable of being converted into an active form by the glutamate carboxypeptidase.

Embodiments of the present invention will now be described by way of example and not limitation with reference to the accompanying figures. However various further aspects and embodiments of the present invention will be apparent to those skilled in the art in view of the present disclosure.

"and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

### Brief Description of the Figures

**Figure 1****.** CPG2-catalysed conversion of N-(4-[bis(2-iodoethyl)amino]phenoxycarbonyl)-L-glutamic acid to N-(4-[bis(2-iodoethyl)amino]phenol.
**Figure 2****.** Scheme of tumour cell death triggered by gene-directed enzyme prodrug therapy (GDEPT) following viral expression of a prodrug-activating enzyme in the tumour cells and administration of prodrug.
**Figure 3****.** Molecular biology steps in the generation of VV-CPG2.
**Figure 4****.** CPG2 expression in head & neck FaDu xenografts after intratumoural (IT) or intravenous (IV) administration of AdV-hTert or VV-CPG2 vectors.
**Figure 5****.** AdV-hTert in GDEPT of FaDu head & neck cancer xenografts. AdV-hTert delivered intratumouraly (IT). Tumour volume (mm³)vs time (A); Percentage survival vs time (B). The top plot of (A) represents the control group.
**Figure 6****.** VV-CPG2 in GDEPT of FaDu head & neck cancer xenografts. VV-CPG2 delivered intratumouraly (IT). Tumour volume (mm³)vs time(A); Percentage survival vs time(B). The lowest plot of (A) and the upper-right plot of (B) represent the GDEPT group.
**Figure 7****.** AdV-hTert in GDEPT of FaDu head & neck cancer xenografts. AdV-hTert delivered intravenously (IV). Tumour volume (mm³)vs time(A); Percentage survival vs time(B). The top plot of (A) and the lowest plot of (B) represent the control group.
**Figure 8****.** VV-CPG2 in GDEPT of FaDu head & neck cancer xenografts. VV-CPG2 delivered intravenously (IV). Tumour volume vs time (mm³) (A) ; Percentage survival vs time (B). The lowest plot of (A) and the upper-right plot of (B) represent the GDEPT group.
**Figure 9****.** VV-CPG2 in GDEPT of FaDu head & neck cancer xenografts in combination with radiotherapy (RT). VV-CPG2 delivered intratumouraly (IT). Tumour volume vs time(mm³) (A); Percentage survival vs time(B). The lower plot of (A) (extending to 160 days) and the upper-right plot of (B) represent the RT+GDEPT group.
**Figure 10****.** VV-CPG2 in GDEPT of FaDu head & neck cancer xenografts in combination with radiotherapy (RT). VV-CPG2 delivered intravenously (IV), tumour volume vs time. The lowest plot represents the RT+GDEPT group.
**Figure 11****.** MTS viability assay of WM266.4 cells treated with ZD2767P prodrug, either untransduced (RHS y-axis) or transduced with VV-CPG2 at MOI 0.1 (LHS y-axis).
**Figure 12****.** MTS viability assay of FaDu cells treated with ZD2767P prodrug, either untransduced (RHS y-axis) or transduced with VV-CPG2 at MOI 0.1 (LHS y-axis).
**Figure 13****.** MTS viability assay of SW620 cells treated with ZD2767P prodrug, either untransduced (RHS y-axis) or transduced with VV-CPG2 at MOI 0.1 (LHS y-axis).
**Figure 14****.** MTS viability assay of A549 cells treated with ZD2767P prodrug, either untransduced (RHS y-axis) or transduced with VV-CPG2 at MOI 0.1 (LHS y-axis).
**Figure 15****.** CPG2 expression in Lung A549 xenografts after intravenous (IV) administration of VV-CPG2 vectors (A); and CPG2 expression in colorectal SW620 xenografts after intravenous (IV) administration of VV-CPG2 vectors (B).
**Figure 16****.** Tumour/tissue CPG2 expression after intravenous (IV) administration of VV-CPG2 vectors in mice with head & neck FaDu xenografts(A); colorectal SW620 xenografts (B); and lung cancer A549 xenografts (C). Activity is expressed in unit activity per gram (for solid tissues) and unit activity per ml (for plasma).
**Figure 17****.** VV-CPG2 in GDEPT of lung cancer A549 xenografts. VV-CPG2 delivered intravenously (IV). Tumour volume vs time (mm³) (A); Percentage survival vs time (B). The lowest plot of (A) and the upper plot of (B) represent the GDEPT group.

### Detailed Description

### Gene-directed enzyme prodrug therapy (GDEPT)

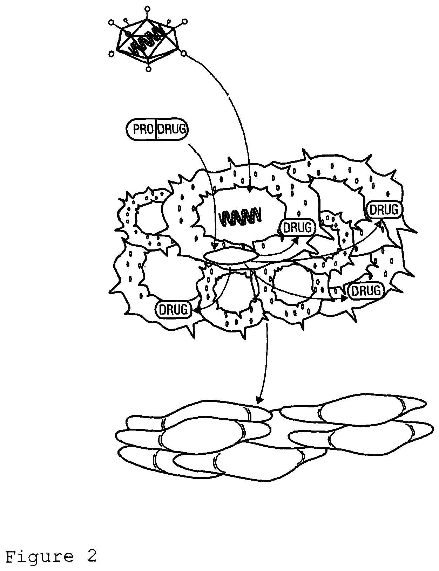

GDEPT is a two-step treatment for tumours. Foreign, prodrug-activating enzymes are delivered to, and expressed in, target cells where they can activate subsequently administered non-toxic prodrugs to form active drugs. Figure 2 shows a scheme of a GDEPT system in which, in a first step, a tumour-infecting virus is used to deliver the gene expressing the prodrug-activating enzyme. In the second step, a prodrug is administered that can be activated to form a toxic drug by the enzyme that has been expressed in the tumour. The prodrug-activating enzyme gene should be expressed exclusively, or with a relatively high ratio, in tumour cells compared with normal tissues and blood, and should achieve a sufficient concentration for clinical benefit. After gene delivery, prodrug administration should be delayed to permit protein expression in the targeted cells. The catalytic activity of the expressed enzyme should be sufficient for activation of the prodrug. Since expression of the prodrug-activating enzymes will not occur in all cells of a targeted tumour in vivo, a bystander cytotoxic effect is beneficial, whereby the prodrug is cleaved to an active drug that kills not only tumour cells but also neighbouring non-expressing tumour cells. This means that expression in less than 100% of tumour cells can still result in killing of all tumour cells. The prodrug-activating enzyme is usually expressed intracellularly, but it may be a membrane-tethered variant that is directed to the cell surface.

### Enzyme systems

A number of different prodrug-activating enzymes have been used in GDEPT therapy. For example, cytosine deaminase, HSV thymidine kinase and cytochrome P450 have been used in GDEPT (Hedley et. al., Nat. Rev. Can. 7, 870-879, 2007). Any one of these enzymes can be used with vaccinia virus according to the present invention.

The GDEPT system developed by the present inventors uses the enzyme carboxypeptidase G2 (CPG2) from Pseudomonas strain RS16. Peptidases are a class of enzymes which act upon a substrate to cleave a peptide (amide) bond; carboxypeptidases cleave peptide bonds at the carboxy-terminal of a protein or peptide.

In some embodiments of the present invention, the prodrug-activating enzyme is a carboxypeptidase, which converts the prodrug into an active drug by removing a protecting group from the prodrug. The prodrug-activating enzyme may be a glutamate carboxypeptidase, e.g., CPG1 or CPG2, which preferentially cleave glutamate and glutamate analogues from the prodrug.

The preferred enzyme is truncated carboxypeptidase CPG2 from Pseudomonas strain RS16 (disclosed in WO88/07378), having the sequence shown in SEQ ID NO:2, although full-length CPG2, membrane anchored CPG2, other mutants, orthologues, homologues or variants of CPG2 may also be used. In the context of this application, a CPG2 homologue is an enzyme that shares a common ancestry with CPG2, which is able to convert a prodrug to an active drug at substantially the same rate as the CPG2 of SEQ ID NO:2. In the context of this application, CPG2 mutants have an amino acid sequence consisting of at least 80%, at least 90%, at least 95%, at least 98%, or at least 99% of SEQ ID NO:2 and also optionally comprise a further amino acid sequence derived from another protein. Variants may comprise an amino acid sequence which has at least 80%, at least 90%, at least 95%, at least 98%, or at least 99% identity to SEQ ID NO:2. Variants may be encoded by a nucleotide sequence which has at least 80%, at least 90%, at least 95%, at least 98%, or at least 99% identity to SEQ ID NO:1. Sequence comparison may be made over the full-length of the relevant sequence shown herein, or may more preferably be over a contiguous sequence of about or greater than about 20, 25, 30, 33, 40, 50, 67, 133, 167, 200, 233, 267, 300, 333, or more amino acids or nucleotide triplets, compared with the relevant amino acid sequence or nucleotide sequence as the case may be. Alterations to the sequence will be such that the enzyme retains its ability to convert a prodrug to an active drug at substantially the same rate as the native enzyme. In this context, "substantially the same rate" will desirably be within 1 order of magnitude, and preferably from about 50-fold e.g. about 2-fold less to 2, 5 or 10 fold more.

CPG2 variants used according to the invention may be engineered to be directed to the cell membrane (i.e. membrane-tethered CPG2), as described in WO 01/085960, to enable prodrugs with poor intracellular availability to be activated in the extracellular space.

Alternatively, other bacterial carboxypeptidase enzymes may be used, e.g., CPG2 enzymes from other Pseudomonas species such as Pseudomonas aeruginosa, Pseudomonas cepacia, Pseudomonas fluorescens, Pseudomonas putida, Pseudomonas syringae, Pseudomonas savastanoi. Preferred carboxypeptidases will have one or more of the following properties: immunological cross-reactivity with an antibody reactive to the polypeptide for which the sequence given in SEQ ID NO:2; sharing an epitope with the polypeptide for which the amino acid sequence is shown in SEQ ID NO:2 (as determined for example by immunological cross-reactivity between the two polypeptides); a biological activity which is inhibited by an antibody raised against the polypeptide whose sequence is shown in SEQ ID NO: 2; ability to release L-glutamic acid from benzoic acid mustard prodrugs. Alteration of sequence may change the nature and/or level of activity and/or stability of the carboxypeptidase enzyme.

A preferred substrate for CPG2 is an L-glutamic acid group, linked to an aromatic ring via an amidic, carbamic, or ureidic linkage. Glutamic acid analogs are also acceptable substrates. For example, L-glutamic acid modified at the γ-carbon (e.g., with an amide, -CONH₂, instead of an acid, -COOH) also serves as a suitable substrate for CPG2.

### Prodrugs

A range of prodrugs have been used in GDEPT therapy. For example, 5-fluorocytosine has been used in cytosine deaminase GDEPT systems, ganciclovir has been used in HSV thymidine kinase GDEPT systems and cyclophosphamide has been used in Cytochrome P450 GDEPT systems (Hedley et. al., Nat. Rev. Can. 7, 870-879, 2007). Any one of these prodrug/enzyme systems can be used with vaccinia virus according to the present invention.

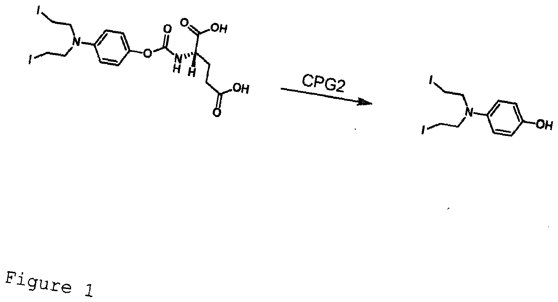

The GDEPT system that the present inventors have developed uses the enzyme carboxypeptidase G2 (CPG2) to activate prodrugs such as benzoic acid nitrogen mustard prodrugs, aniline nitrogen mustard prodrugs and phenol nitrogen mustard prodrugs such as ZD2767P (N-(4-[bis(2-iodoethyl)amino]phenoxycarbonyl)-L-glutamic acid) and salts thereof. CPG2 cleaves the carbamate linkage of ZD2767P to release L-glutamic acid, a carbon dioxide molecule and the DNA alkylating nitrogen mustard N-(4-[bis(2-iodoethyl)amino]phenol, which is a potent cytotoxic agent. Figure 1 shows the CPG2-catalysed conversion of ZD2767P to produce N-(4-[bis(2-iodoethyl)amino]phenol.

Nitrogen mustards are related to sulfur mustard, (ClCH₂CH₂)₂S, the "mustard gas" used during the First World War. Nitrogen mustards have the general formula (ClCH₂CH₂)₂NR. In vivo, each 2-chloroethyl side-chain undergoes an intramolecular cyclisation with the release of a chloride ion. The resulting highly reactive ethylene immonium derivative can interact with DNA and other molecules, for example, as an alkylating and/or crosslinking agent. Nitrogen mustards are useful, for example, in the treatment of proliferative conditions, such as cancer. Nitrogen mustard analogues, in which the chloro group is replaced by other groups, such as other halogens (e.g., bromo and iodo, as exemplified by ZD2767P) and other good leaving groups (e.g., sulfonates, such as mesyloxy, -OSO₂Me) are also known, and are included in the class denoted "nitrogen mustards". Nitrogen mustards may conveniently be grouped according to the group R. For example, two groups are phenolic nitrogen mustards and anilinic nitrogen mustards.

In GDEPT systems, nitrogen mustards or other cytotoxic drugs are provided in the form of a prodrug, which has a protecting group linked to the cytotoxic moiety. In most cases, the protecting group will be cleaved as a whole from the prodrug. However, it is also possible for the enzyme to cleave or simply alter part of the protecting group, resulting in a partially cleaved or altered protecting group which is unstable, leading to spontaneous removal of the remainder of the group.

A range of prodrugs which are suitable substrates for CPG2 have been described previously (WO 94/002450, WO 04/020400). A partial structure of typical substrates is; -aromatic-ring-X-CO-NH-Glu, where X is -NH-, -O- or -CH2, and Glu is glutamic acid or a glutamic acid analogue.

Besides ZD2767P, other nitrogen mustard prodrugs which can be used according to the present invention in CPG2 GDEPT systems include:
(S)-2-(4-[bis(2-chloroethyl)amino]phenoxycarbonylamino)-4-(1H-1,2,3,4-tetrazol-5-yl)butyric acid and salts thereof;
N-(4-[bis(2-chloroethyl)amino]-3-fluorophenylcarbamoyl)-L-glutamic acid and salts thereof;
N-(4-[bis(2-chloroethyl)amino]phenylcarbamoyl)-L-glutamic acid and salts thereof; and
N-(4-[bis(2-chloroethyl)amino]phenoxycarbonyl)-L-glutamic acid and salts thereof.

WO 04/020400 describes "self-immolative" CPG2 substrates, which are defined as compounds that, following an activation process, generate an unstable intermediate that releases the active drug in a number of subsequent steps. Hence, CPG2 is able to activate these self-immolative prodrugs by cleaving or simply altering part of the prodrug protecting group, resulting in a partially cleaved or altered protecting group which is unstable, leading to spontaneous removal of the remainder of the group.

The skilled person would understand that any of the prodrugs described herein, or any other compounds that can be used with the prodrug-activating enzymes considered herein, can be used according to the invention without undue burden.

### Vaccinia virus

Vaccinia virus is a member of the poxviridae family of large DNA viruses, which typically have a genome of between 130-300 kbp in size, which is encoded on a single length of linear doublestranded DNA. To accommodate the relatively large quantity of genetic material, poxviruses have a large capsid having dimensions of around 200nm by 300nm. Their large DNA carrying capacity allows poxviruses to be engineered to deliver multiple transgenes and/or transgenes of a large size.

Poxvirus virions take different forms at different stages of their lifecycle. The extracellular enveloped virion is the infectious particle which binds and enters the cell. The extracellular enveloped virion (EEV) has a viral core within a capsid particle, which is sheathed in an outer membrane. The outer membrane is removed upon cell entry, thus defining a first part of the two-part process of uncoating of the EEV. In the second part of the uncoating process, the viral core containing the viral DNA is released into the cytoplasm.

Vaccinia genes are expressed in the cytoplasm, and this process takes place in several stages including early gene expression, intermediate gene expression and late gene expression. Genes which are necessary for genome replication, e.g. viral DNA-dependent RNA polymerase, are early phase genes. Genes which modulate the host anti-viral response and promote virus replication are also predominantly expressed during the early phase of infection. In contrast, viral structural components are mainly expressed during the late phase, with a relatively small group of genes thought to be important in activating late gene expression being expressed in the intermediate phase. Structural genes assemble in the cytosol to form the intracellular mature virion (IMV), which becomes coated with two membranes by the Golgi apparatus to form the intracellular enveloped virion (IEV).

The IEV is translocated along the cytoskeletal microtubules to the cell periphery where it fuses with the cell membrane to form the cell-associated enveloped virion (CEV), which is released as EEV when the cell is lysed, causing cell death. Vaccinia virus according to the present invention will predominantly be in the form of EEV, however even the intracellular forms of the vaccinia virion are known to be infectious and are also considered to form a part of the invention.

The vaccinia virus genome contains many virulence genes which function to modulate the host anti-viral response or disrupt the host cell cycle in order to promote intracellular conditions suitable for viral replication. For example, vaccinia viruses contain genes which express thymidine kinase, interferon-binding proteins, serine proteinase inhibitors and epidermal growth factor receptor (EGFR)-binding growth factors. Mutations to members of each of these classes of vaccinia virulence genes in order to enhance vaccinia selectivity for infecting tumour cells have been previously reported (Kirn et al., Nat. Rev. Can., 9, 64-71, 2009). Vaccinia virus having a deletion of the type I IFN-binding binding protein selectively replicates in tumours with a loss of interferon response. Deletion of the serine proteinase inhibitors SPI-1 and SPI-2, which interfere with host antiviral defence, results in vaccinia that preferentially replicate in transformed cells. Deletion of vaccinia thymidine kinase leads to dependence of the virus on cellular thymidine kinase expression, which is constitutively expressed in the majority of cancers, regardless of proliferation status (Kirn et al., Nat. Rev. Can., 9, 64-71, 2009).

Vaccinia viruses have been shown to efficiently infect and kill a range of tumour types including lung cancer, cervix cancer and CNS cancer (A Ascierto et al., BMC Cancer, 11:451, 2011) and melanoma, ovarian cancer, kidney cancer, breast cancer, leukemia, non-small cell lung carcinoma, colon cancer, brain tumour, prostate cancer (Parato et al., Mol. Ther., 20(4), 749-758, 2012).

The tumour selectivity of a virus can be measured or quantified by using standard techniques. For example, viral DNA copy number in tumour samples can be compared with viral DNA copy number in non-tumour samples by performing the polymerase chain reaction (PCR) on each sample, thereby allowing the skilled person to quantitatively assess the degree of tumour selectivity of a virus. The skilled person would understand that alternative techniques such as viral protein-specific Enzyme-Linked Immunosorbent Assay (ELISA) may also be used to determine and compare viral load in in tumour and non-tumour samples.

### VV-CPG2

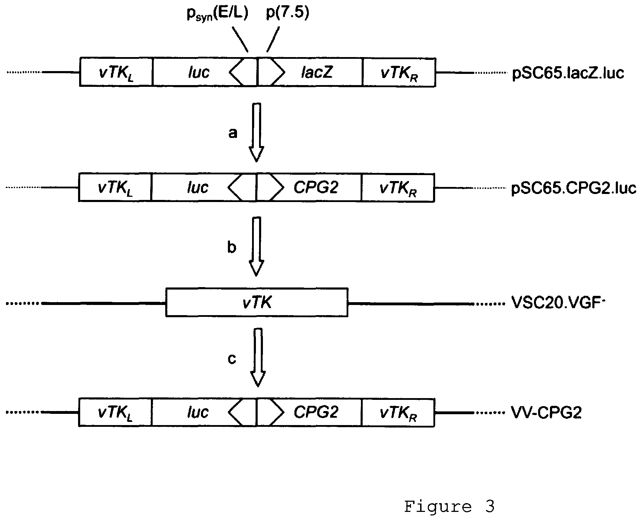

The present inventors have found that vaccinia viruses which are modified to express CPG2 are capable of eliciting a potent antitumour response in vivo when used in GDEPT protocols together with a suitable prodrug. DNA expressing CPG2 under the control of the vaccinia p7.5 promoter was inserted into the vaccinia thymidine kinase (TK) gene, resulting in vaccinia virus which has inactivated thymidine kinase. The vaccinia growth factor (VGF) gene, which is homologous to epidermal growth factor, was also deleted. Furthermore, DNA expressing luciferase under the control of the vaccinia p_{syn}(E/L) promoter was also inserted to allow infected cells to be conveniently identified. Figure 3 shows the orientation of luciferase and CPG2 in the TK gene of VV-CPG2.

### Examples

### Materials and Methods

### Cell lines, parental virus and plasmids

Authenticated FaDu, SW620 and WM266.4 were obtained from American Type Culture Collection (LGC Promochem). FaDu cells are a telomerase-positive human carcinoma cell line. The cell lines used for virus production and characterization, CV1 cells (from American Type Culture Collection), 143B TK- (authenticated by STR profiling) and the parental virus, VSC20.VGF- and the shuttle vector, pSC65.lacZ.luc, based on pSC65 (Chakrabarti et al., 1997), were supplied by Jennerex Inc. Construction of the plasmid containing the CPG2 sequence and AdV-hTert have been previously described (Marais et al., 1996)(Schepelmann et al, 2005, 2007).

### Construction of VV-CPG2

Figure 2 shows a schematic of the molecular biology steps in the cloning of the VV-CPG2 plasmid.

pSC65.lacZ.luc was digested with XhoI and BamHI to remove the lacZ sequence and religated with the polylinker sequence TCGAGACGCGTGATATCATGCATACATGTCACGTGGAATTCACTAGTG (top) and GATCCACTAGTGAATTCCACGTGACATGTATGCATGATATCACGCGTC (bottom) to produce pSC65.polylinker.luc, which was digested with MluI and SpeI. pEF.CPG2 was modified to contain the adaptor-Kozak sequence ACGCGTGCCGCCACC (top) and GGTGGCGGCACGCGT (bottom) immediately upstream of the CPG2 ATG start codon, giving pEF.adaptor.CPG2, which was digested with MluI and XbaI. The linearized shuttle plasmid, pSC65.polylinker.luc, was religated with the excised CPG2 sequence to give pSC65.CPG2.luc, in which CPG2 and luc are downstream of the vaccinia promoters p7.5 and psyn(E/L), respectively.

Recombination of pSC65.CPG2.luc with VSC20.VGF- and plaque purification of recombinants was performed according to standard protocols (Earl et al., 1998). Briefly, CV1 cells were infected with VSC20.VGF- and subsequently transfected with pSC65.CPG2.luc using Lipofectamine (Invitrogen). Bioluminescence indicated cellular colocalisation of vaccinia transcription factors and the luciferase gene. Recombinants were selected by three rounds of plaque purification in 143B TK- cells in the presence of bromodeoxyuridine, and validated by transgene region sequencing, bioluminescence and CPG2 activity assay (Stribbling et al., 1997). Recombinant virus was expanded to working stocks on CV1 cells, purified by sucrose gradient centrifugation and quantified by plaque titration. The engineered virus containing deletions of tk and vgf and insertions of cpg2 and luc was designated VV-CPG2. Insert fidelity was confirmed by dye-terminator cycle sequencing.

### Infection of tumour cell lines with VV-CPG2

The following cell lines were tested for susceptibility to transduction with VV-CPG2;
- SW620 and HT29: colorectal cancer cell lines
- A549: lung cancer
- FaDu: head and neck cancer
- WM266.4: melanoma
- MDA-MB-231: Breast cancer

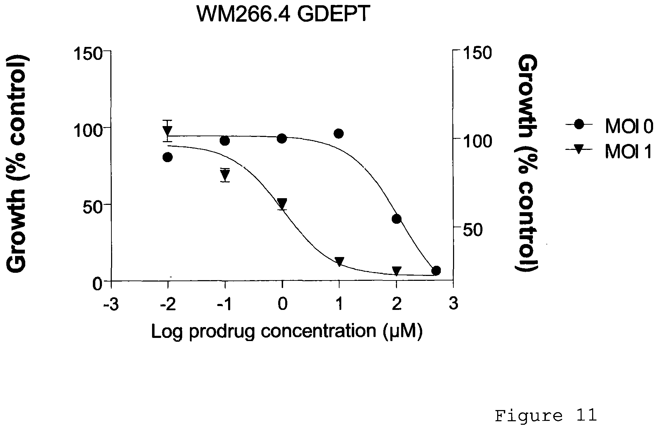

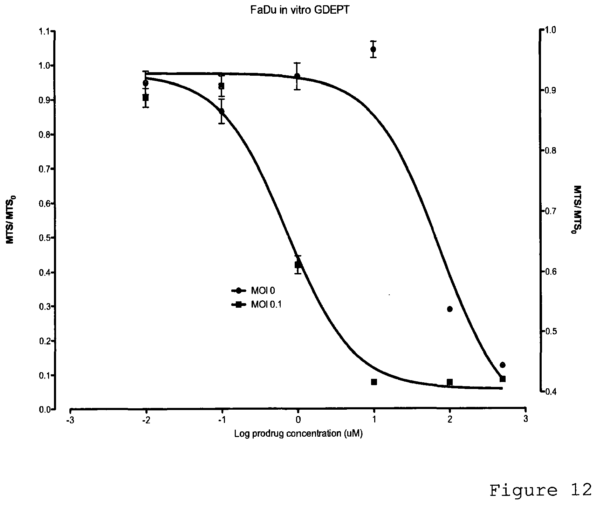

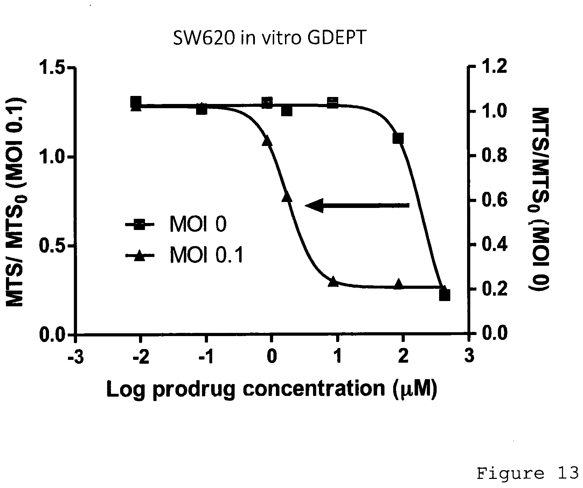

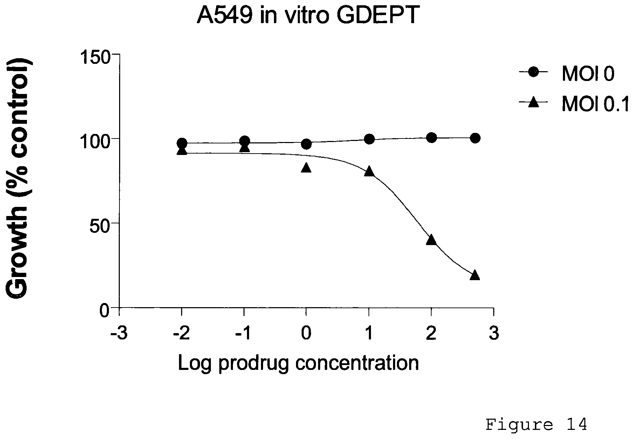

Each cell tumour cell line tested was found to express CPG2 following infection with VV-CPG2. For example, A549 cells, MDA-MB-231 cells and HT29 cells each transduced with VV-CPG2 at a multiplicity of infection (MOI) of 0.1 had CPG2 activity of 1 unit/mg (U/mg), 1.2 U/mg or 1.1 U/mg at 72 hours post-transduction, respectively. The reduction of cell viability in WM226.4 cells, FaDu cells, SW620 and A549 cells is shown in figures 11 to 14. In each case, cell viability was measured by MTS assay. A reduction of the EC50 of ZD2767P in cells transduced with VV-CPG2 at MOI of 0.1 is: 114-fold (WM226.4 cells); 119-fold (SW620 cells); and 103-fold (FaDu cells).

Hence, in each case, transduction with VV-CPG2 at MOI = 0.1 results in over 100-fold increase in sensitivity to ZD2767P.

### In vivo studies

All animal work was compliant with Personal and Project UK Home Office License restrictions and UKCCCR guidelines (Workman, 1998). CD1 nu/nu athymic female mice were obtained from Charles River at 5-7 weeks of age, and allowed to acclimatize for one week prior to study. A specific pathogen-free environment was maintained. Animals had unlimited access to food and water and were housed at no more than five per cage. To establish tumour xenografts, cells in exponential growth phase were injected into the subcutaneous compartment of the right flank. FaDu cells were injected at 10⁶ cells per animal in PBS. Interventions were initiated at 14 or 15 days after cell instillation in the presence of established tumours. A549 cells were injected at 10⁷ cells per animal in PBS and Matrigel, in a 1:1 ratio. Interventions were initiated when tumours were approximately 100mm³. SW620 cells were injected at 10⁶ cells per animal in PBS.

Animals were sacrificed by cervical dislocation when the mean tumour diameter exceeded 15 mm or any single diameter reached 17 mm, or in the presence of progressive tumour ulceration, as directed by project license conditions.

For intratumoural studies AdV-hTert was administered at 4x10⁸ plaque forming units/kg (PFU/kg) in 0.2ml/20g bodyweight, while VV-CPG2 was administered 4x10⁷ PFU/kg by intratumoural injection in a total volume of 20 µl vehicle, using a systematic method adapted from published precedent (Bazan-Peregrino et al., 2008). For intravenous studies, AdV-hTert was administered at 4x10⁹ PFU/kg in 0.2ml/20g bodyweight by tail vein injection. VV-CPG2 was also administered at 4x10⁹ PFU/kg in 0.2ml/20g bodyweight by tail vein injection. ZD2767P was administered by intraperitoneal (IP) injection at 150-300 mg/kg in three divided doses at one hour intervals. Four to six prodrug administrations were performed over 4-8 weeks, guided by toxicity monitoring. In control groups, substance administrations were substituted by appropriate vehicles. Tumour caliper measurements were taken twice weekly and volumes calculated using the formula length x width x height x π/6. Tissue CPG2 activities were quantified by an indirect endpoint assay as previously described (Stribbling et al., 1997). Briefly, samples were homogenized and incubated with methotrexate for 30 minutes when the reaction was terminated. The concentration of the degradation product, DAMPA, was measured by high performance liquid chromatography. The end product concentration was converted to CPG2 activity by reference to standard curves, generated for each analysis using an alternative kinetic assay. Irradiation was performed using a Gammacell 40 Exactor (Best Theratronics, Ottawa, Canada), adapted to deliver unilateral localised tumour irradiation from a single caesium-137 source at 662 kV. Offline dosimetry was performed using Gafchromic EBT film (International Specialty Products, Vertec Scientific, UK), giving a current dose rate of 0.6 Gy per minute. In control groups, substance administrations were substituted by appropriate vehicles, and mock-irradiation was performed by sedation and placement of animals in the treatment chamber for similar duration.

### Results

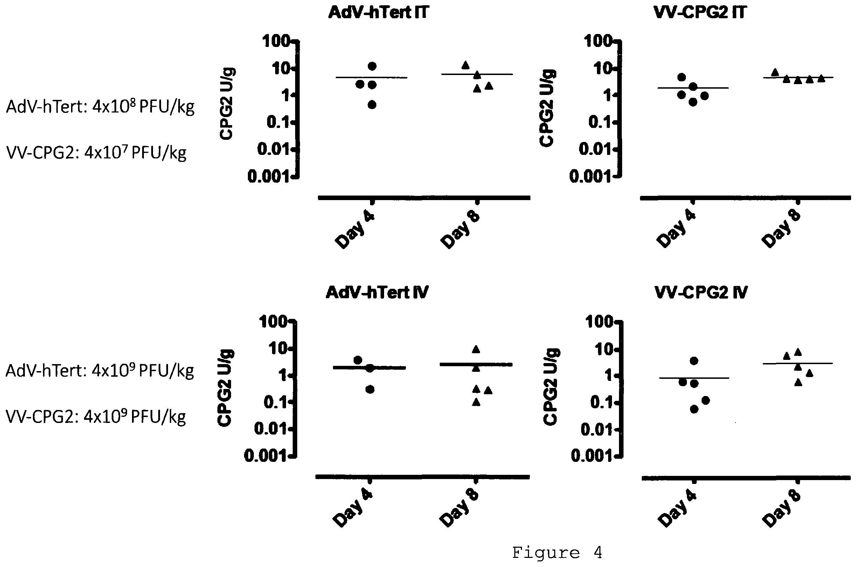

Figure 4 shows concentration of the CPG2 enzyme in tumours following intratumoural (IT) or intravenous (IV) injection of AdV-hTERT or VV-CPG2 in the FaDu human tumour xenograft model. CPG2 enzyme levels in the FaDu human tumour xenograft model, measured in units per gram of tumour tissue (CPG2 U/g), were determined via an activity endpoint assay, as described below. Figure 4 shows that similar levels of CPG2 activity are achieved following IV injection of VV-CPG2 in the FaDu human tumour xenograft model compared with AdV-hTERT administered at the same dose of 4 x 10⁹ plaque forming units/kg. For IT administration in the FaDu human tumour xenograft model, a 4 x 10⁷ pfu/kg VV-CPG2 is sufficient to achieve a similar level of CPG2 activity to that achieved by a dosage of 4 x 10⁸ of AdV-hTERT in the FaDu human tumour xenograft model.

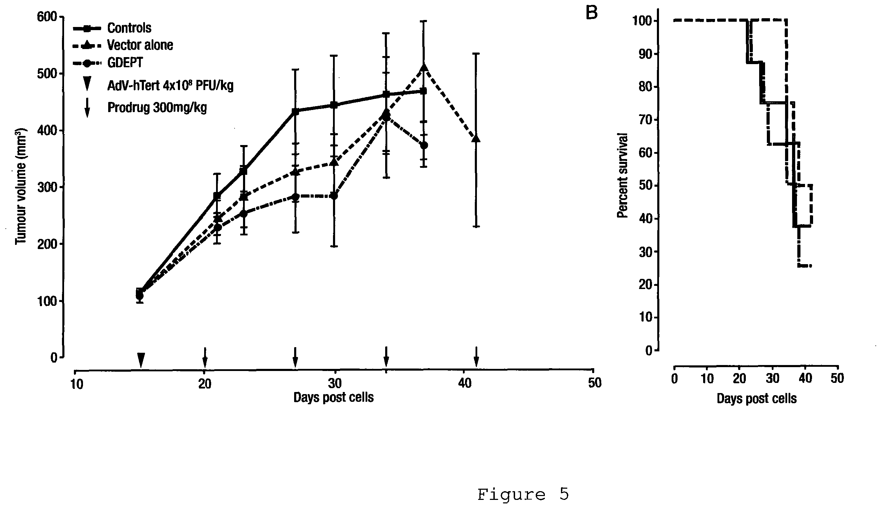

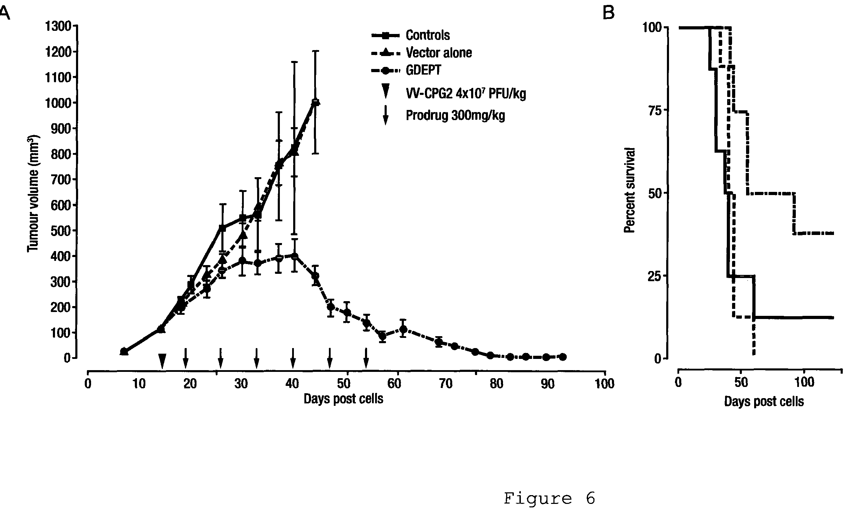

Figures 5 and 6 show, in the FaDu human tumour xenograft model, the effect of GDEPT on tumour volume and animal survival following IT administration of the AdV-hTERT vector or the VV-CPG2 vector, respectively. Figure 5A shows that AdV-hTERT-mediated GDEPT does not effect a reduction in tumour volume. The tumour volume of AdV-hTERT-GDEPT treated animals is not significantly different from the tumour volume of control animals or animals treated with the AdV-hTERT vector alone (without subsequent prodrug administration). Figure 5B shows that animal survival following AdV-hTERT-mediated GDEPT is not extended; if anything, survival time is shortened. In contrast, in the FaDu human tumour xenograft model, Figure 6A shows that VV-CPG2-mediated GDEPT results in a reduction in tumour volume which is significant and persistent over time, with tumour volume tending to zero in surviving animals. Figure 6B shows that animal survival following VV-CPG2-mediated GDEPT is extended, with approximately 35% of VV-CPG2-GDEPT animals surviving for over 100 days.

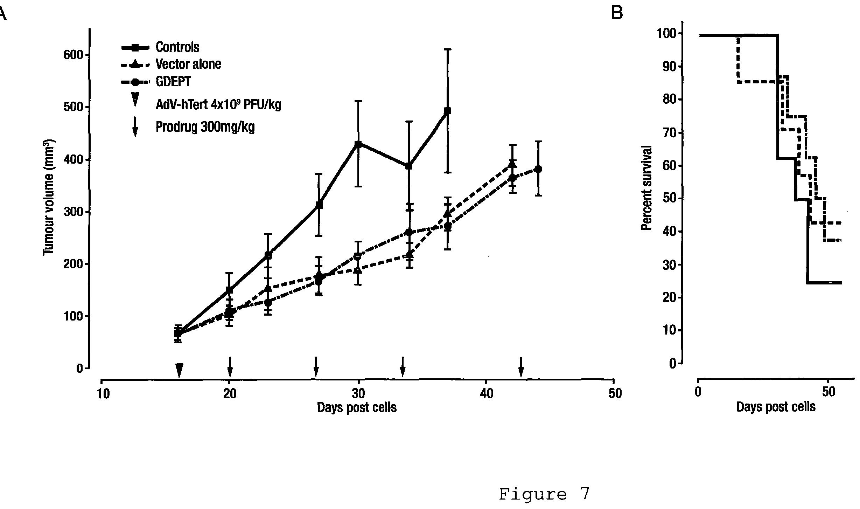

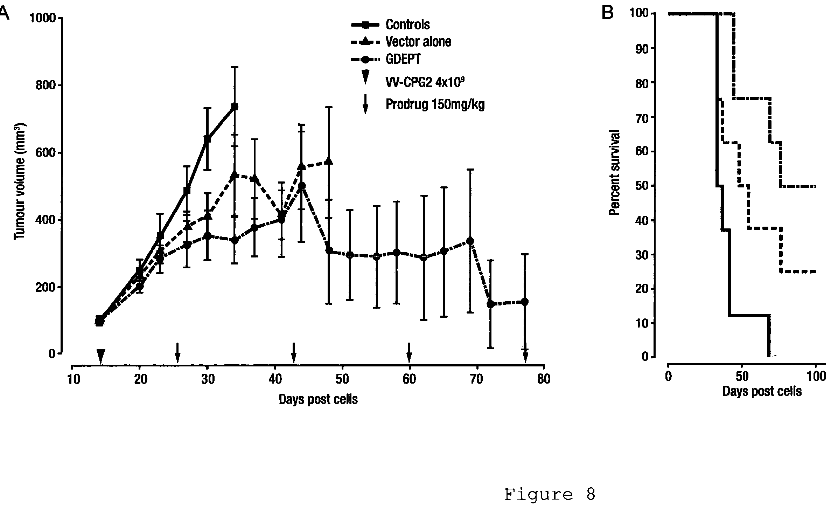

Figures 7 and 8 show, in the FaDu human tumour xenograft model, the effect of GDEPT on tumour volume and animal survival following IV administration of the AdV-hTERT vector and the VV-CPG2 vector, respectively. Figure 7A shows that AdV-hTERT-mediated GDEPT does not effect a reduction in tumour volume. The tumour volume of AdV-hTERT-GDEPT treated animals is not significantly different from the tumour volume of control animals or animals treated with the AdV-hTERT vector alone (without subsequent prodrug administration). Figure 7B shows that animal survival following AdV-hTERT-mediated GDEPT is only slightly extended. Figure 8A, in the FaDu human tumour xenograft model, shows that VV-CPG2-mediated GDEPT results in a reduction in tumour volume over time. Figure 8B shows that animal survival following VV-CPG2-mediated GDEPT is extended, with approximately 50% VV-CPG2-GDEPT animals surviving for over 100 days.

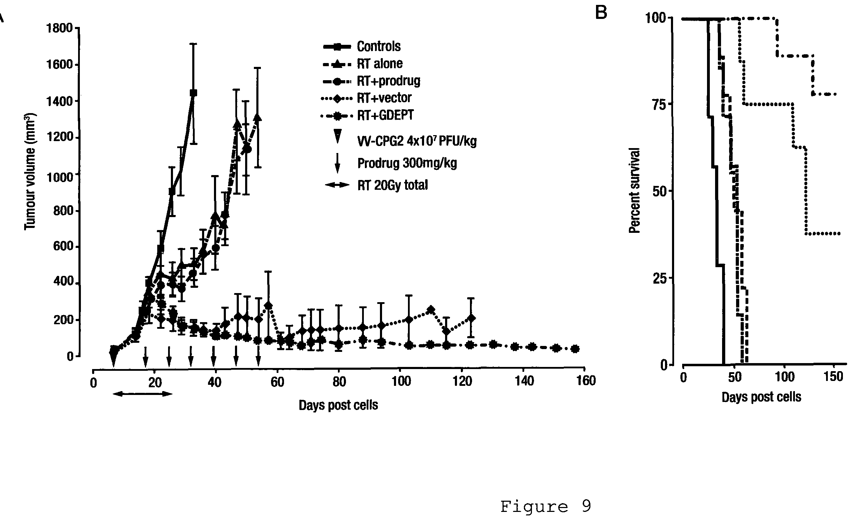

Figure 9 shows the combination efficacy between VV-CPG2-mediated GDEPT and radiotherapy (RT) in the FaDu human tumour xenograft model. VV-CPG2 was delivered intratumouraly. All animals, except controls, received a 20Gy radiation dose. Figure 9A shows that RT in combination with the vector alone results in a stabilisation of tumour volume over time and that RT in combination with VV-CPG2-mediated GDEPT results in reduced tumour volumes, which tend to zero. Figure 9B shows that over 75% of animals that received VV-CPG2-mediated GDEPT in combination with radiotherapy survived for over 150 days.

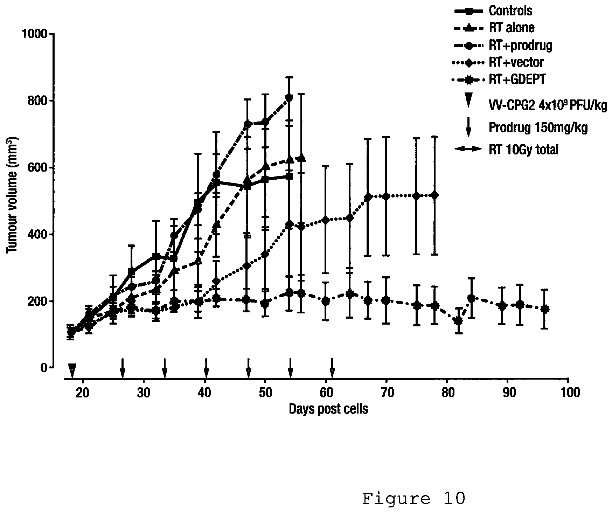

Figure 10 shows that VV-CPG2-mediated GDEPT in combination with radiotherapy (RT) results in stable tumour volume when VV-CPG2 is delivered intravenously. All animals, except controls, received a 10Gy radiation dose.

Other combinations of anticancer therapy may be envisaged as forming a part of the invention. For example, cell checkpoint inhibitors such as Chk1, known to sensitise tumours to cytotoxic agents, may be used alongside the GDEPT systems described herein for cancer therapies.

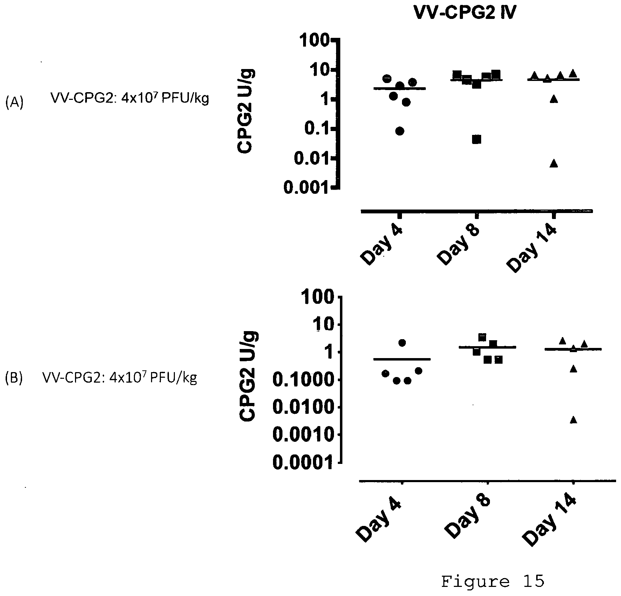

Figure 15 shows concentration of the CPG2 enzyme in (A) Lung A549 tumours and (B) colorectal SW620 tumours following intravenous (IV) injection of VV-CPG2. CPG2 enzyme levels, measured in units per gram of tumour tissue (CPG2 U/g), were determined via an activity endpoint assay. Figure 15 shows that similar levels of CPG2 activity are achieved in lung cancer models as with the head & neck cancer model shown in figure 4.

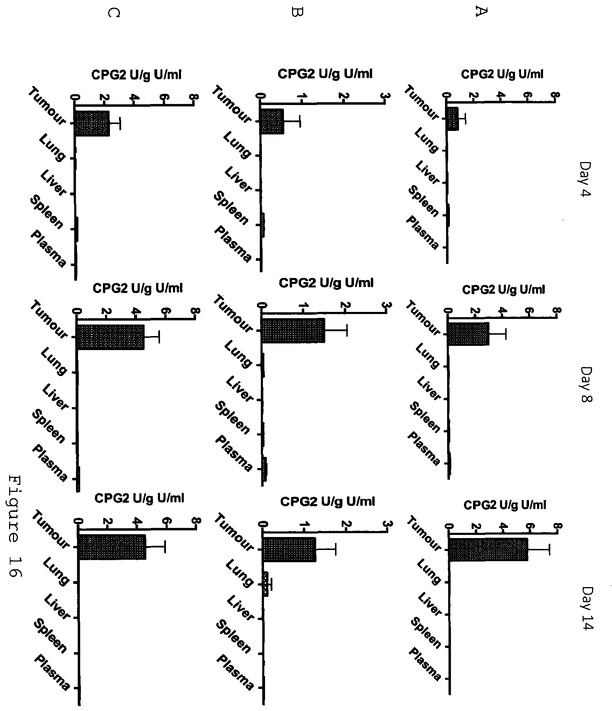

Figure 16 illustrates the excellent tumour specificity of the VV-CPG2 vaccinia vector for head & neck FaDu tumours (A), colorectal SW620 tumours (B), and lung cancer A549 tumours (C).

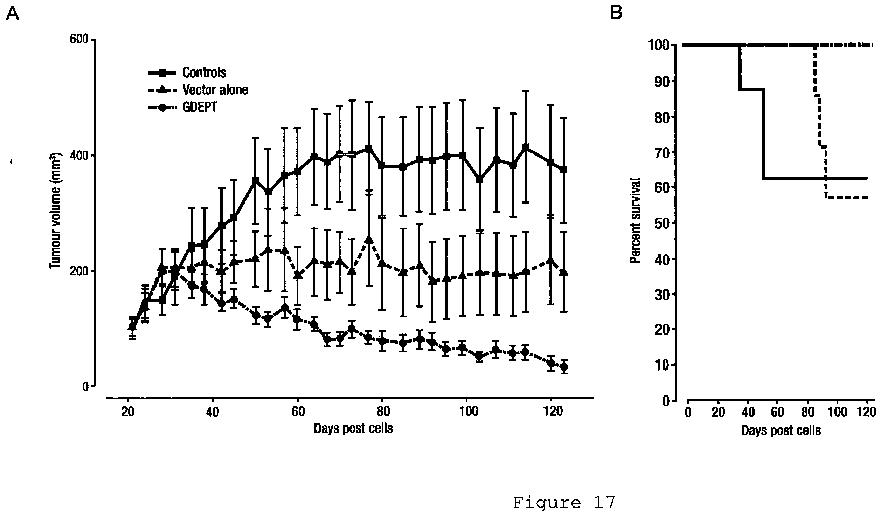

Figure 17A shows the effect of GDEPT on tumour volume and animal survival following IV administration of the VV-CPG2 to animals with lung cancer A549 tumours. The tumour volume of control animals or animals treated with the VV-CPG2 vector alone (without subsequent prodrug administration) was only slightly reduced (central plot). This antitumoural effect is enhanced when the prodrug is also administered (lower plot). The ZD2767P prodrug was administered starting 4 days after VV-CPG2 injection by intraperitoneal (IP) injection at 150mg/kg in three divided doses at one hour intervals. Six prodrug administrations were performed over 7 weeks. In control groups, substance administrations were substituted by appropriate vehicles.

Figure 17B shows that animal survival following VV-CPG2-mediated GDEPT results in 100% survival (top line) compared with approximately 40% mortality of the control animals after 50 days.

### Sequence Listing

SEQ ID NO:1 - CPG2* DNA sequence:

atggccggatccgccctggcccagaagcgcgacaacgtgctgttccaggcagctaccgac
gagcagccggccgtgatcaagacgctggagaagctggtcaacatcgagaccggcaccggt
gacgccgagggcatcgccgctgcgggcaacttcctcgaggccgagctcaagaacctcggc
ttcacggtcacgcgaagcaagtcggccggcctggtggtgggcgacaacatcgtgggcaag
atcaagggccgcggcggcaagaacctgctgctgatgtcgcacatggacaccgtctacctc
aagggcattctcgcgaaggccccgttccgcgtcgaaggcgacaaggcctacggcccgggc
atcgccgacgacaagggcggcaacgcggtcatcctgcacacgctcaagctgctgaaggaa
tacggcgtgcgcgactacggcaccatcaccgtgctgttcaacaccgacgaggaaaagggt
tccttcggctcgcgcgacctgatccaggaagaagccaagctggccgactacgtgctctcc
ttcgagcccaccagcgcaggcgacgaaaaactctcgctgggcacctcgggcatcgcctac
gtgcaggtcaacatcaccggcaaggcctcgcatgccggcgccgcgcccgagctgggcgtg
aacgcgctggtcgaggcttccgacctcgtgctgcgcacgatgaacatcgacgacaaggcg
aagaacctgcgcttcaactggaccatcgccaaggccggcaacgtctcgaacatcatcccc
gccagcgccacgctgaacgccgacgtgcgctacgcgcgcaacgaggacttcgacgccgcc
atgaagacgctggaagagcgcgcgcagcagaagaagctgcccgaggccgacgtgaaggtg
atcgtcacgcgcggccgcccggccttcaatgccggcgaaggcggcaagaagctggtcgac
aaggcggtggcctactacaaggaagccggcggcacgctgggcgtggaagagcgcaccggc
ggcggcaccgacgcggcctacgccgcgctctcaggcaagccagtgatcgagagcctgggc
ctgccgggcttcggctaccacagcgacaaggccgagtacgtggacatcagcgcgattccg
cgccgcctgtacatggctgcgcgcctgatcatggatctgggcgccggcaaggaattcctc
gagatcgattagactagtctaga

SEQ ID NO:2 - CPG2* amino acid sequence:

MAGSALAQKRDNVLFQAATDEQPAVIKTLEKLVNIETGTGDAEGIAAAGNFLEAELKNLG
FTVTRSKSAGLVVGDNIVGKIKGRGGKNLLLMSHMDTVYLKGILAKAPFRVEGDKAYGPG
IADDKGGNAVILHTLKLLKEYGVRDYGTITVLFNTDEEKGSFGSRDLIQEEAKLADYVLS
FEPTSAGDEKLSLGTSGIAYVQVNITGKASHAGAAPELGVNALVEASDLVLRTMNIDDKA
KNLRFNWTIAKAGNVSNIIPASATLNADVRYARNEDFDAAMKTLEERAQQKKLPEADVKV
IVTRGRPAFNAGEGGKKLVDKAVAYYKEAGGTLGVEERTGGGTDAAYAALSGKPVIESLG
LPGFGYHSDKAEYVDISAIPRRLYMAARLIMDLGAGKEFLEID*TSL

SEQ ID NO:3 - pSC65.luc.lacZ:

agcttttgcgatcaataaatggatcacaaccagtatctcttaacgatgttcttcgcagat
gatgattcatttttttaagtatttggctagtcaagatgatgaaatcttcattatctgatat
attgcaaatcactcaatatctagactttctgttattattattgatccaatcaaaaaataa
attagaagccgtgggtcattgttatgaatctctttcagaggaatacagacaattgacaaa
attcacagactttcaagattttaaaaaactgtttaacaaggtccctattgttacagatgg
aagggtcaaacttaataaaggatatttgttcgactttgtgattagtttgatgcgattcaa
aaaagaatcctctctagctaccaccgcaatagatcctgttagatacatagatcctcgtcg
caatatcgcattttctaacgtgatggatatattaaagtcgaataaagtgaacaataatta
attctttattgtcatcatgaacggcggacatattcagttgataatcggccccatgttttc

aggtaaaagtacagaattaattagacgagttagacgttatcaaatagctcaatataaatg
cgtgactacaaaatattctaacgataatagatacggaacggactatggacgcatgataa
gaataattttgaagcattggaagcaactaaactatgtgatgtcttggaatcaattacaga
tttctccgtgataggtatcgatgaaggacagttctttccagacattgttgaattagatcg
ataaaaattaattaattacccgggtaccacatttgtagagagttttacttgctttaaaaa
acctcccacacctccccctgaacctgaaacataaaatgaatgcaattgttgttgttaact
tgtttattgcagcttacaatggttacaaataaagcaatagcatcacaaatttcacaaata
aagcatttttttcactgcattctagttgtggtttgtccaaactcatcaatgtatcttatc
atgtctgctcgaagcggccggccgccccgactccagaattacacggcgatcttttccgcc
cttcttgggcctttatgagagatctctctgatttttcttggcgtcgagttttccgggtaa
gacctttcggtactttcgtccacaaacacaactcctccgcgcaactttttcgcggttgt
tacttgactggcgacgtaatccacgatctctttttccgtcatcgtctttccgtgctccaa
aacaacaacggcggcgggaagttcaccggcgtcatcgtcgggaagacctgcgacacctgc
gtcgaagatgttggggtgttggagcaagatggattccaattcagcgggagccacctgata
gcctttgtacttaatcagagacttcaggcggtcaacgatgaagaagtgttcgtcttcgtc
ccagtaagctatgtctccagaatgtagccatccatccttgtcaatcaaggcgttggtcgc
ttccggattgtttacataaccggacataatcataggacctctcacacacagttcgcctct
ttgattaacgcccagcgttttcccggtatccagatccacaaccttcgcttcaaaaaatgg
aacaactttaccgaccgcgcccggtttatcatccccctcgggtgtaatcagaatagctga
tgtagtctcagtgagcccatatccttgcctgatacctggcagatggaacctcttggcaac
cgcttccccgacttccttagagaggggagcgccacccgtttgccatttcgtgtaaattag
ataaatcgtatttgtcaatcagagtgcttttggcgaagaaggagaataggttggcacca
gcagcgcactttgaatcttgtaatcctgaaggctcctcagaaacagctcttcttcaatct
atacattatgacgactcgaaatccacatatcaaatatccgagtgtagtaaacattccaaa
accgtgatggaatggaacaacacttaaaatcgcagtatccggaatgatttgattgccaaa
aataggatctctggcatgcgagaatctcacgcaggcagttctatgaggcagagcgacacc
tttaggcagaccagtagatccagaggagttcatgatcagtgcaattgtcttgtccctatc
gaaggactctggcacaaaatcgtattcattaaaaccgggaggtagatgagatgtgacgaa
cgtgtacatcgactgaaatccctggtaatccgttttagaatccatgataataatttttg
gatgattgggagctttttttgcacgttcaaaatttttgcaacccctttttggaaacgaa
caccacggtaggctgcgaaatgcccatactgttgagcaattcacgttcattataaatgtc
gttcgcgggcgcaactgcaactccgataaataacgcgcccaacaccggcataaagaattg
aagagagttttcactgcatacgacgattctgtgatttgtattcagcccatatcgtttcat
agcttctgccaaccgaacggacatttcgaagtactcagcgtaagtgatgtccacctcgat
atgtgcatctgtaaaagcaattgctccaggaaccagggcgtatctcttcatagccttatg
cagttgctctccagcggttccatcttccagcggatagaatggcgccgggcctttcttat
gtttttggcgtcttccatggtaccaggcctagatctgtcgacttcgagcttatttatatt
ccaaaaaaaaaaaataaaatttcaattttaagctttcactaattccaaacccacccgct
ttttatagtaagtttttcacccataaataataaatacaataattaatttctcgtaaaagt
agaaaatatattctaatttattgcacggtaaggaagtagatcataactcgagcatgggag
atcccgtcgttttacaacgtcgtgactgggaaaaccctggcgttacccaacttaatcgcc
ttgcagcacatccccctttcgccagctggcgtaatagcgaagaggcccgcaccgatcgcc
cttcccaacagttgcgcagcctgaatggcgaatggcgctttgcctggtttccggcaccag
aagcggtgccggaaagctggctggagtgcgatcttcctgaggccgatactgtcgtcgtcc
cctcaaactggcagatgcacggttacgatgcgcccatctacaccaacgtaacctatccca
ttacggtcaatccgccgtttgttcccacggagaatccgacgggttgttactcgctcacat
ttaatgttgatgaaagctggctacaggaaggccagacgcgaattatttttgatggcgtta
actcggcgtttcatctgtggtgcaacgggcgctgggtcggttacggccaggacagtcgtt
tgccgtctgaatttgacctgagcgcatttttacgcgccggagaaaaccgcctcgcggtga
tggtgctgcgttggagtgacggcagttatctggaagatcaggatatgtggcggatgagcg

gcattttccgtgacgtctcgttgctgcataaaccgactacacaaatcagcgatttccatg
ttgccactcgctttaatgatgatttcagccgcgctgtactggaggctgaagttcagatgt
gcggcgagttgcgtgactacctacgggtaacagtttctttatggcagggtgaaacgcagg
tcgccagcggcaccgcgcctttcggcggtgaaattatcgatgagcgtggtggttatgccg
atcgcgtcacactacgtctcaacgtcgaaaacccgaaactgtggagcgccgaaatcccga
atctctatcgtgcggtggttgaactgcacaccgccgacggcacgctgattgaagcagaag
cctgcgatgtcggtttccgcgaggtgcggattgaaaatggtctgctgctgctgaacggca
agccgttgctgattcgaggcgttaaccgtcacgagcatcatcctctgcatggtcaggtca
tggatgagcagacgatggtgcaggatatcctgctgatgaagcagaacaactttaacgccg
tgcgctgttcgcattatccgaaccatccgctgtggtacacgctgtgcgaccgctacggcc
tgtatgtggtggatgaagccaatattgaaacccacggcatggtgccaatgaatcgtctga
ccgatgatccgcgctggctaccggcgatgagcgaacgcgtaacgcgaatggtgcagcgcg
atcgtaatcacccgagtgtgatcatctggtcgctggggaatgaatcaggccacggcgcta
atcacgacgcgctgtatcgctggatcaaatctgtcgatccttcccgcccggtgcagtatg
aaggcggcggagccgacaccacggccaccgatattatttgcccgatgtacgcgcgcgtgg
atgaagaccagcccttcccggctgtgccgaaatggtccatcaaaaaatggctttcgctac
ctggagagacgcgcccgctgatcctttgcgaatacgcccacgcgatgggtaacagtcttg
gcggtttcgctaaatactggcaggcgtttcgtcagtatccccgtttacaggcggcttcg
tctgggactgggtggatcagtcgctgattaaatatgatgaaaacggcaacccgtggtcgg
cttacggcggtgattttggcgatacgccgaacgatcgccagttctgtatgaacggtctgg
tctttgccgaccgcacgccgcatccagcgctgacggaagcaaaacaccagcagcagtttt
tccagttccgtttatccgggcaaaccatcgaagtgaccagcgaatacctgttccgtcata
gcgataacgagctcctgcactggatggtggcgctggatggtaagccgctggcaagcggtg
aagtgcctctggatgtcgctccacaaggtaaacagttgattgaactgcctgaactaccgc
agccggagagcgccgggcaactctggctcacagtacgcgtagtgcaaccgaacgcgaccg
catggtcagaagccgggcacatcagcgcctggcagcagtggcgtctggcggaaaacctca
gtgtgacgctccccgccgcgtcccacgccatcccgcatctgaccaccagcgaaatggatt
tttgcatcgagctgggtaataagcgttggcaatttaaccgccagtcaggctttctttcac
agatgtggattggcgataaaaaacaactgctgacgccgctgcgcgatcagttcacccgtg
caccgctggataacgacattggcgtaagtgaagcgacccgcattgaccctaacgcctggg
tcgaacgctggaaggcggcgggccattaccaggccgaagcagcgttgttgcagtgcacgg
cagatacacttgctgatgcggtgctgattacgaccgctcacgcgtggcagcatcagggga
aaaccttatttatcagccggaaaacctaccggattgatggtagtggtcaaatggcgatta
ccgttgatgttgaagtggcgagcgatacaccgcatccggcgcggattggcctgaactgcc
agctggcgcaggtagcagagcgggtaaactggctcggattagggccgcaagaaaactatc
ccgaccgccttactgccgcctgttttgaccgctgggatctgccattgtcagacatgtata
ccccgtacgtcttcccgagcgaaaacggtctgcgctgcgggacgcgcgaattgaattatg
gcccacaccagtggcgcggcgacttccagttcaacatcagccgctacagtcaacagcaac
tgatggaaaccagccatcgccatctgctgcacgcggaagaaggcacatggctgaatatcg
acggtttccatatggggattggtggcgacgactcctggagcccgtcagtatcggcggaat
tcagctgagcgccggtcgctaccattaccagttggtctggtgtcaaaaataataataacc
gggcaggggggatccttctgtgagcgtatggcaaacgaaggaaaaatagttatagtagcc
gcactcgatgggacatttcaacgtaaaccgtttaataatatttgaatcttattccatta
tctgaaatggtggtaaaactaactgctgtgtgtatgaaatgctttaaggaggcttccttt
tctaaacgattgggtgaggaaaccgagatagaaataataggaggtaatgatatgtatcaa
tcggtgtgtagaaagtgttacatcgactcataatattatatttttatctaaaaaactaa
aaataaacattgattaaattttaatataatacttaaaaatggatgttgtgtcgttagata
aaccgtttatgtattttgaggaaattgataatgagttagattacgaaccagaaagtgcaa
atgaggtcgcaaaaaaactgccgtatcaaggacagttaaaactattactaggagaattat
tttttcttagtaagttacagcgacacggtatattagatggtgccaccgtagtgtatatag

gatctgctcccggtacacatatacgttatttgagagatcatttctataatttaggagtga
tcatcaaatggatgctaattgacggccgccatcatgatcctatttaaatggattgcgtg
atgtgactctagtgactcggttcgttgatgaggaatatctacgatccatcaaaaaacaac
tgcatccttctaagattatttaatttctgatgtgagatccaaacgaggaggaaatgaac
ctagtacggcggatttactaagtaattacgctctacaaaatgtcatgattagtatttaa
accccgtggcgtctagtcttaaatggagatgcccgtttccagatcaatggatcaaggact
tttatatccacacggtaataaaatgttacaacctttgctccttcatattcagctgaaa
tgagattattaagtatttataccggtgagaacatgagactgactcggccgcgttgctgg
cgtttttccataggctccgcccccctgacgagcatcacaaaaatcgacgctcaagtcaga
ggtggcgaaacccgacaggactataaagataccaggcgtttccccctggaagctccctcg
tgcgctctcctgttccgaccctgccgcttaccggatacctgtccgcctttctcccttcgg
gaagcgtggcgctttctcaatgctcacgctgtaggtatctcagttcggtgtaggtcgttc
gctccaagctgggctgtgtgcacgaaccccccgttcagcccgaccgctgcgccttatccg
gtaactatcgtcttgagtccaacccggtaagacacgacttatcgccactggcagcagcca
ctggtaacaggattagcagagcgaggtatgtaggcggtgctacagagttcttgaagtggt
ggcctaactacggctacactagaaggacagtatttggtatctgcgctctgctgaagccag
ttaccttcggaaaaagagttggtagctcttgatccggcaaacaaaccaccgctggtagcg
gtggtttttttgtttgcaagcagcagattacgcgcagaaaaaaaggatctcaagaagatc
ctttgatcttttctacggggtctgacgctcagtggaacgaaaactcacgttaagggattt
tggtcatgagattatcaaaaaggatcttcacctagatccttttaaattaaaaatgaagtt
ttaaatcaatctaaagtatatatgagtaaacttggtctgacagttaccaatgcttaatca
gtgaggcacctatctcagcgatctgtctatttcgttcatccatagttgcctgactccccg
tcgtgtagataactacgatacgggagggcttaccatctggccccagtgctgcaatgatac
cgcgagacccacgctcaccggctccagatttatcagcaataaaccagccagccggaaggg
ccgagcgcagaagtggtcctgcaactttatccgcctccatccagtctattaattgttgcc
gggaagctagagtaagtagttcgccagttaatagtttgcgcaacgttgttgccattgctg
caggcatcgtggtgtcacgctcgtcgtttggtatggcttcattcagctccggttcccaac
gatcaaggcgagttacatgatcccccatgttgtgcaaaaaagcggttagctccttcggtc
ctccgatcgttgtcagaagtaagttggccgcagtgttatcactcatggttatggcagcac
tgcataattctcttactgtcatgccatccgtaagatgcttttctgtgactggtgagtact
caaccaagtcattctgagaatagtgtatgcggcgaccgagttgctcttgcccggcgtcaa
cacgggataataccgcgccacatagcagaactttaaaagtgctcatcattggaaaacgtt
cttcggggcgaaaactctcaaggatcttaccgctgttgagatccagttcgatgtaaccca
ctcgtgcacccaactgatcttcagcatcttttactttcaccagcgtttctgggtgagcaa
aaacaggaaggcaaaatgccgcaaaaaagggaataagggcgacacggaaatgttgaatac
tcatactcttcctttttcaatattattgaagcatttatcagggttattgtctcatgagcg
gatacatatttgaatgtatttagaaaaataaacaaataggggttccgcgcacatttcccc
gaaaagtgccacctgacgtctaagaaaccattattatcatgacattaacctataaaaata
ggcgtatcacgaggccctttcgtcttcgaataaatacctgtgacggaagatcacttcgca
gaataaataaatcctggtgtccctgttgataccgggaagccctgggccaacttttggcga
aaatgagacgttgatcggcacgtaagaggttccaactttcaccataatgaaataagatca
ctaccgggcgtattttttgagttatcgagattttcaggagctaaggaagctaaaatggag
aaaaaaatcactggatataccaccgttgatatatcccaatggcatcgtaaagaacatttt
gaggcatttcagtcagttgctcaatgtacctataaccagaccgttcag

### References:

1. B. E. Huber, E. A. Austin, C. A. Richards, S. T. Davis, and S. S. Good, Proceedings of the National Academy of Sciences of the United States of America, 1994, 91, 8302-8306.
2. WO 96/03151, C. J. Springer et. al.
3. R. Marais, R. A. Spooner, Y. Light, J. Martin, and C. Springer, Cancer Research, 1996, 56, 4735-4742.
4. L. Chen, L. J. Yu, and D. J. Waxman, Cancer Research, 1997, 57, 4830-4837.
5. S. Schepelmann, L. M. Ogilvie, D. Hedley, F. Friedlos, J. Martin, I. Scanlon, P. Chen, R. Marais, and C. J. Springer, Cancer Research, 2007, 67, 4949-4955.
6. D. Hedley, L. Oglivie, and C. Springer, Nature Reviews Cancer, 2007, 7, 870-879.
7. Y. Jounaidi, J. C. Doloff, and D. J. Waxman, Current Cancer Drug Targets, 2007, 7(3), 285-301.
8. M. Puhlmann, M. Gnant, C. K. Brown, H. R. Alexander, D. L. Bartlett, Human Gene Therapy, 1999, 10, 649-57.
9. J. A. McCart, M. Puhlmann, J. Lee, Gene Therapy, 2000, 7, 1217-23.
10. WO 01/085960, C. J. Springer et. al.
11. WO 94/002450, C. J. Springer et. al.
12. WO 04/020400, C. J. Springer et. al.
13. S. Chakrabarti, J. R. Sisler, and B. Moss, BioTechniques, 1997, 23(6), 1094-1097.
14. D. H. Kirn and S. H. Thorne, Nature Reviews Cancer, 2009, 9, 64-71.
15. S. Schepelmann, P. Hallenbeck, L. M. Ogilvie, D. Hedley, F. Friedlos, J. Martin, I. Scanlon, C. Hay, L. K. Hawkins, R. Marais, and C. J. Springer, Cancer Research, 2005, 65, 5003-5008.
16. P. L. Earl et. al., Current Protocols in Molecular Biology, 1998, 16.16.1 - 16.17.19.
17. S. M. Stribling, J. Martin, R. B. Pedley, J. A. Boden, S. K. Sharma, and C. J. Springer, Cancer Chemother. Pharmacol, 1997, 40, 277-284.
18. M. Bazan-Peregrino, R. C. Carlisle, L. Purdie, and L. W. Seymour, Gene Therapy, 2008, 15, 688-694.
19. M. L. Ascierto, A. Worschech, Z. Yu, S. Adams, J. Reinboth, N. G. Chen, Z. Pos, R. Roychoudhuri, G. Di Pasquale, D. Bedognetti, L. Uccellini, F. Rossano, P. A. Ascierto, D. F. Stroncek, N. P. Restifo, E. Wang, A. A. Szalay, and F. M. Marincola BMC Cancer, 2011, 11:451**.**
20. A. Parato, C. J. Breitbach, F. Le Boeuf, J. Wang, Chris Storbeck, C. Ilkow, Jean-Simon Diallo, T. Falls, J. Burns, V. Garcia, F. Kanji, L. Evgin, K. Hu, F. Paradis, S Knowles, Tae-Ho Hwang, B. C. Vanderhyden, R. Auer, D. H. Kirn and J C Bell, Molecular Therapy, 2012, 20(4), 749-758.

## Claims

1. A vaccinia virus for use in treating a tumour in a subject,
wherein the vaccinia virus expresses a glutamate carboxypeptidase within a cell,
wherein the method includes the administration of a prodrug to the subject, wherein the prodrug is capable of being converted into an active form by the glutamate carboxypeptidase.

2. The vaccinia virus for use in treating a tumour according to claim 1, wherein the vaccinia virus is genetically modified to have enhanced tumour selectivity, optionally wherein the vaccinia virus has one or more inactivated virulence genes, further optionally wherein the one or more inactivated virulence genes is vaccinia thymidine kinase (vTK), yet further optionally wherein the inactivated vTK gene comprises a CPG2 expression cassette insert and/or a luciferase expression cassette insert.

3. The vaccinia virus for use in treating a tumour according to claim 2, wherein the vaccinia virus has one or more inactivated virulence genes which include vaccinia growth factor (VGF).

4. The vaccinia virus for use in treating a tumour according to any one of the preceding claims, wherein the tumour is a solid tumour, optionally wherein the tumour is selected from; head and neck cancer, colorectal cancer, lung cancer, melanoma, or breast cancer, cervix cancer, CNS cancer, ovarian cancer, kidney cancer, leukaemia, brain tumour, or prostate cancer, further optionally wherein the tumour is selected from; head and neck cancer, colorectal cancer, lung cancer, melanoma, or breast cancer.

5. The vaccinia virus for use in treating a tumour according to any one of the preceding claims, wherein the method includes the administration of the vaccinia virus by intravenous or intratumoural injection

6. The vaccinia virus for use in treating a tumour according to any one of the preceding claims, wherein the method includes the administration of radiotherapy to the subject

7. The vaccinia virus for use in treating a tumour according to any one of the preceding claims, wherein the method includes the administration of a further anticancer agent, optionally wherein the further anticancer agent is a Chk1 inhibitor.

8. The vaccinia virus for use in treating a tumour according to any one of the preceding claims, wherein the glutamate carboxypeptidase is CPG2 or a mutant, variant or homologue thereof, optionally wherein the CPG2 comprises an amino acid sequence which has at least 80% identity to SEQ ID NO: 2.

9. A vaccinia virus which expresses a glutamate carboxypeptidase within a cell, wherein the glutamate carboxypeptidase is CPG2 which has at least 80% identity to SEQ ID NO: 2, optionally wherein the vaccinia virus comprises a CPG2 expression cassette insert in a vaccinia virus virulence gene, further optionally wherein the vaccinia virus virulence gene is vaccinia thymidine kinase (vTK).

10. A two component system for gene directed enzyme prodrug therapy which comprises: (a) a vaccinia virus according to claim 9; and
(b) a prodrug which can be converted into an active drug by a glutamate carboxypeptidase.

11. An *in vitro* method of killing a neoplastic cell, the method comprising treating the neoplastic cell with: a vaccinia virus according to claim 9; and, a prodrug which is capable of being converted into an active form by a glutamate carboxypeptidase.

12. The vaccinia virus for use in treating a tumour according to any one of claims 1-8, the vaccinia virus according to claim 9, the two component system of claim 10, or the method of killing a neoplastic cell according to claim 11, wherein the prodrug comprises a glutamic acid group or a glutamic acid analogue group, wherein the glutamic acid group or the glutamic acid analogue is connected via N to a linker which is connected to a cytotoxic agent.

13. The vaccinia virus for use in treating a tumour according to claim 12, the vaccinia virus according to claim 12, the two component system according to claim 12, or the method of killing a neoplastic cell according to claim 12, wherein the linker comprises a functional group selected from: carbonyl, carbamate, urea, and equivalents.

14. The vaccinia virus for use in treating a tumour according to claim 12 or 13, the vaccinia virus according to claim 12 or 13, the two component system according to claim 12 or 13, or the method of killing a neoplastic cell according to claim 12 or 13, wherein the cytotoxic agent comprises a nitrogen mustard or a nitrogen mustard analogue.

15. The vaccinia virus for use in treating a tumour according to claim 14, the vaccinia virus according to claim 14, the two component system according to claim 14, or the method of killing a neoplastic cell according to claim 14, wherein the prodrug is; N-(4-[bis(2-iodoethyl)amino]phenoxycarbonyl)-L-glutamic acid or a salt thereof.

## Patentansprüche

1. Vacciniavirus zur Verwendung bei der Behandlung eines Tumors in einem Individuum, wobei das Vacciniavirus eine Glutamat-Carboxypeptidase in einer Zelle exprimiert,
wobei das Verfahren das Verabreichen eines Prodrugs an das Individuum umfasst, wobei das Prodrug durch die Glutamat-Carboxypeptidase in eine aktive Form umgewandelt werden kann.

2. Vacciniavirus zur Verwendung bei der Behandlung eines Tumors nach Anspruch 1, wobei das Vacciniavirus genetisch modifiziert ist, um eine verstärkte Tumorselektivität aufzuweisen, wobei das Vacciniavirus gegebenenfalls ein oder mehrere inaktivierte Virulenzgene aufweist, wobei das eine oder die mehreren inaktivierten Virulenzgene gegebenenfalls außerdem Vaccinia-Thymidinkinase (vTK) ist oder sind, wobei das inaktivierte vTK-Gen gegebenenfalls außerdem ein CPG2-Expressionskassetteninsert und/oder ein Luciferase-Expressionskassetteninsert umfasst.

3. Vacciniavirus zur Verwendung bei der Behandlung eines Tumors nach Anspruch 2, wobei das Vacciniavirus ein oder mehrere inaktivierte Virulenzgene aufweist, das oder die Vaccinia-Wachstumsfaktor (VGF) umfasst oder umfassen.

4. Vacciniavirus zur Verwendung bei der Behandlung eines Tumors nach einem der vorangegangenen Ansprüche, wobei der Tumor ein fester Tumor ist, wobei der Tumor gegebenenfalls aus Kopf- und Halskrebs, Kolorektalkrebs, Lungenkrebs, Melanom oder Brustkrebs, Gebärmutterhalskrebs, Zentralnervensystem-Krebs, Eierstockkrebs, Nierenkrebs, Leukämie, Hirntumor oder Prostatakrebs ausgewählt ist, wobei der Tumor außerdem gegebenenfalls aus Kopf- und Halskrebs, Kolorektalkrebs, Lungenkrebs, Melanom oder Brustkrebs ausgewählt ist.

5. Vacciniavirus zur Verwendung bei der Behandlung eines Tumors nach einem der vorangegangenen Ansprüche, wobei das Verfahren die Verabreichung des Vacciniavirus durch intravenöse oder intratumorale Injektion umfasst.

6. Vacciniavirus zur Verwendung bei der Behandlung eines Tumors nach einem der vorangegangenen Ansprüche, wobei das Verfahren die Verabreichung einer Strahlentherapie an das Individuum umfasst.

7. Vacciniavirus zur Verwendung bei der Behandlung eines Tumors nach einem der vorangegangenen Ansprüche, wobei das Verfahren die Verabreichung eines weiteren Antikrebsmittels umfasst, wobei das weitere Antikrebsmittel gegebenenfalls ein Chk1-Hemmer ist.

8. Vacciniavirus zur Verwendung bei der Behandlung eines Tumors nach einem der vorangegangenen Ansprüche, wobei die Glutamat-Carboxypeptidase CPG2 oder eine Mutante, Variante oder ein Homolog davon ist, wobei CPG2 gegebenenfalls eine Aminosäuresequenz umfasst, die zumindest 80 % Identität mit SEQ ID NO: 2 aufweist.

9. Vacciniavirus, das eine Glutamat-Carboxypeptidase in einer Zelle exprimiert, wobei die Glutamat-Carboxypeptidase CPG2 ist, das zumindest 80 % Identität mit SEQ ID NO: 2 aufweist, wobei das Vacciniavirus gegebenenfalls ein CPG2-Expressionskassetteninsert in einem Vacciniavirus-Virulenzgen umfasst, wobei das Vacciniavirus-Virulenzgen außerdem gegebenenfalls Vaccinia-Thymidinkinase (vTK) ist.

10. Zweikomponentensystem zur gengesteuerten Enzym-Prodrug-Therapie, die Folgendes umfasst:
(a) ein Vacciniavirus nach Anspruch 9; und
(b) ein Prodrug, das durch Glutamat-Carboxypeptidase in ein aktives Arzneimittel umgewandelt werden kann.

11. In-vitro-Verfahren zum Abtöten einer neoplastischen Zelle, wobei das Verfahren das Behandeln der neoplastischen Zelle mit Folgendem umfasst: einem Vacciniavirus nach Anspruch 9; und einem Prodrug, das durch eine Glutamat-Carboxypeptidase in eine aktive Form umgewandelt werden kann.

12. Vacciniavirus zur Verwendung bei der Behandlung eines Tumors nach einem der Ansprüche 1 bis 8, Vacciniavirus nach Anspruch 9, Zweikomponentensystem nach Anspruch 10 oder Verfahren zum Abtöten einer neoplastischen Zelle nach Anspruch 11, wobei das Prodrug eine Glutaminsäuregruppe oder eine Glutaminsäureanalogon-Gruppe umfasst, wobei die Glutaminsäuregruppe oder das Glutaminsäureanalogon über N an einen Linker gebunden ist, der an ein zytotoxisches Mittel gebunden ist.

13. Vacciniavirus zur Verwendung bei der Behandlung eines Tumors nach Anspruch 12, Vacciniavirus nach Anspruch 12, Zweikomponentensystem nach Anspruch 12 oder Verfahren zum Abtöten einer neoplastischen Zelle nach Anspruch 12, wobei der Linker eine funktionelle Gruppe umfasst, die aus Carbonyl, Carbamat, Harnstoff und Äquivalenten ausgewählt ist.

14. Vacciniavirus zur Verwendung bei der Behandlung eines Tumors nach Anspruch 12 oder 13, Vacciniavirus nach Anspruch 12 oder 13, Zweikomponentensystem nach Anspruch 12 oder 13 oder Verfahren zum Abtöten einer neoplastischen Zelle nach Anspruch 12 oder 13, wobei das zytotoxische Mittel Stickstofflost oder ein Stickstofflostanalogon umfasst.

15. Vacciniavirus zur Verwendung bei der Behandlung eines Tumors nach Anspruch 14, Vacciniavirus nach Anspruch 14, Zweikomponentensystem nach Anspruch 14 oder Verfahren zum Abtöten einer neoplastischen Zelle nach Anspruch 14, wobei das Prodrug N-(4-[Bis-(2-iodethyl)amino]phenoxycarbonyl)-L-glutaminsäure oder ein Salz davon ist.

## Revendications

1. Virus de la vaccine à utiliser dans le traitement d'une tumeur chez un sujet,
dans lequel le virus de la vaccine exprime une glutamate carboxypeptidase dans une cellule,
dans lequel le procédé comprend l'administration d'un promédicament au sujet, dans lequel le promédicament est capable d'être converti en une forme active par la carboxypeptidase glutamate.

2. Virus de la vaccine à utiliser dans le traitement d'une tumeur selon la revendication 1, dans lequel le virus de la vaccine est modifié génétiquement pour avoir une sélectivité tumorale améliorée, facultativement dans lequel le virus de la vaccine a un ou plusieurs gènes de virulence inactivé(s), en outre facultativement dans lequel les un ou plusieurs gènes de virulence inactivé(s) sont la thymidine kinase de la vaccine (vTK), en outre facultativement dans laquelle le gène de vTK inactivé comprend un insert de cassette d'expression de CPG2 et/ou un insert de cassette d'expression de luciférase.

3. Virus de la vaccine à utiliser dans le traitement d'une tumeur selon la revendication 2, dans lequel le virus de la vaccine a un ou plusieurs gènes de virulence inactivé(s) qui incluent le facteur de croissance de la vaccine (VGF).

4. Virus de la vaccine à utiliser dans le traitement d'une tumeur selon l'une quelconque des revendications précédentes, dans lequel la tumeur est une tumeur solide, facultativement dans lequel la tumeur est choisie parmi ; cancer de la tête et du cou, cancer colorectal, cancer du poumon, mélanome, ou cancer du sein, cancer du col de l'utérus, cancer du SNC, cancer de l'ovaire, cancer du rein, leucémie, tumeur cérébrale ou cancer de la prostate, en outre facultativement dans lequel la tumeur est choisie parmi ; cancer de la tête et du cou, cancer colorectal, cancer du poumon, mélanome ou cancer du sein.

5. Virus de la vaccine à utiliser dans le traitement d'une tumeur selon l'une quelconque des revendications précédentes, dans lequel le procédé comprend l'administration du virus de la vaccine par injection intraveineuse ou intratumorale.

6. Virus de la vaccine à utiliser dans le traitement d'une tumeur selon l'une quelconque des revendications précédentes, dans lequel le procédé comprend l'administration de radiothérapie au sujet.

7. Virus de la vaccine à utiliser dans le traitement d'une tumeur selon l'une quelconque des revendications précédentes, dans lequel le procédé comprend l'administration d'un agent anticancéreux supplémentaire, facultativement dans lequel l'agent anticancéreux supplémentaire est un inhibiteur de Chk1.

8. Virus de la vaccine à utiliser dans le traitement d'une tumeur selon l'une quelconque des revendications précédentes, dans lequel la glutamate carboxypeptidase est la CPG2 ou un mutant, variant ou homologue de celle-ci, facultativement dans lequel la CPG2 comprend une séquence d'acides aminés qui a au moins 80 % d'identité avec SEQ ID NO : 2.

9. Virus de la vaccine qui exprime une glutamate carboxypeptidase dans une cellule, dans lequel la glutamate carboxypeptidase est la CPG2 qui a au moins 80 % d'identité à SEQ ID NO : 2, facultativement dans lequel le virus de la vaccine comprend un insert de cassette d'expression de CPG2 dans un gène de virulence du virus de la vaccine, en outre facultativement dans lequel le gène de virulence du virus de la vaccine est la thymidine kinase de la vaccine (vTK).

10. Système à deux composants pour une thérapie par promédicament enzymatique dirigée qui comprend :
(a) un virus de la vaccine selon la revendication 9 ; et
(b) un promédicament qui peut être converti en un médicament actif par une glutamate carboxypeptidase.

11. Procédé *in vitro* de destruction d'une cellule néoplasique, le procédé comprenant le traitement de la cellule néoplasique avec : un virus de la vaccine selon la revendication 9 ; et, un promédicament qui est capable d'être converti en une forme active par une glutamate carboxypeptidase.

12. Virus de la vaccine à utiliser dans le traitement d'une tumeur selon l'une quelconque des revendications 1 à 8, virus de la vaccine selon la revendication 9, système à deux composants selon la revendication 10, ou procédé de destruction d'une cellule néoplasique selon la revendication 11, où le promédicament comprend un groupe acide glutamique ou un groupe analogue d'acide glutamique, où le groupe acide glutamique ou l'analogue d'acide glutamique est connecté via N à un lieur qui est connecté à un agent cytotoxique.

13. Virus de la vaccine à utiliser dans le traitement d'une tumeur selon la revendication 12, virus de la vaccine selon la revendication 12, système à deux composants selon la revendication 12, ou procédé de destruction d'une cellule néoplasique selon la revendication 12, où le lieur comprend un groupe fonctionnel choisi parmi : carbonyle, carbamate, urée, et équivalents.

14. Virus de la vaccine à utiliser dans le traitement d'une tumeur selon la revendication 12 ou 13, virus de la vaccine selon la revendication 12 ou 13, système à deux composants selon la revendication 12 ou 13, ou procédé de destruction d'une cellule néoplasique selon la revendication 12 ou 13, où l'agent cytotoxique comprend une moutarde à l'azote ou un analogue de moutarde à l'azote.

15. Virus de la vaccine à utiliser dans le traitement d'une tumeur selon la revendication 14, virus de la vaccine selon la revendication 14, système à deux composants selon la revendication 14, ou procédé de destruction d'une cellule néoplasique selon la revendication 14, où le promédicament est : acide N-(4-[bis(2-iodoéthyl)amino]phénoxycarbonyl)-L-glutamique ou un sel de celui-ci.
